# EUROPEAN PATENT APPLICATION

(11) **EP 4 335 472 A1**
(43) Date of publication of application: **13.03.2024**
(21) Application number: 21945666.2
(22) Date of filing: 29.07.2021
(51) Int. Cl.: A61M 5/20, A61M 5/148

(54) **NEEDLE INSERTION ASSISTANT AND MEDICAL DEVICE**

(30) Priority: 16.06.2021 CN 202110668202
(71) Applicant: Suzhou Reveda Medical Biotech Co., Ltd., Suzhou, Jiangsu 215200 (CN)
(72) Inventor: YAN, Ping, Suzhou, Jiangsu 215200 (CN); CHEN, Binwen, Suzhou, Jiangsu 215200 (CN); QU, Qiuyu, Suzhou, Jiangsu 215200 (CN); LIU, Long, Suzhou, Jiangsu 215200 (CN); HUANG, Yuan, Suzhou, Jiangsu 215200 (CN)
(74) Representative: Patentship Patentanwaltsgesellschaft mbH
(86) International application number: PCT/CN2021/109124
(87) International publication number: WO 2022/262085

(57) **Abstract**

The present application provides a needle insertion assistant and a medical device including the needle insertion assistant. The needle insertion assistant includes: a housing assembly defining a first inner lumen axially extending therethrough; a pusher assembly disposed within the first inner lumen and including an inner core and an energy storage element, the inner core configured to be axially movable, the energy storage element disposed between the inner core and the housing assembly; and a drive assembly. The needle insertion assistant is configured such that the drive assembly is configured to drive the inner core to move from distal to proximal to cause the energy storage element to store elastic potential energy. The drive assembly is further configured to cause the energy storage element to release the stored elastic potential energy to drive the inner core to move from proximal to distal.

## Description

### TECHNICAL FIELD

The present invention relates to the field of medical devices and, in particular, to a needle insertion assistant and a medical device.

### BACKGROUND

In the medical field, needle insertion assistants are used in various applications.

Compliance of patients in need of drug delivery through injection needles may be reduced due to pain brought about by piercing of such needles. Microneedle patches provide a pain-free or substantially pain-free alternative which can greatly increase patients' compliance, especially that of children and the elderly. These patches have an array of nano-sized microneedles, in which a drug is loaded, or through which a drug can be delivered.

However, in practical transdermal use of such microneedle patches, it would be difficult to achieve simultaneous subcutaneous insertion of all the microneedles with a single palm press action, and one or more additional presses may therefore be necessary. In these multiple press actions, uncontrolled forces may be applied, possibly leading to non-perpendicular piercing of some needles into the skin. Consequently, they may break and the drug loaded therein may fail to deliver its intended therapeutic effect, causing a waste.

### SUMMARY OF THE INVENTION

It is an object of the present invention to provide a needle insertion assistant and a medical device. The needle insertion assistant can provide a large output pushing force, which enables quick subcutaneous insertion of needles.

To this end, the present invention provides a needle insertion assistant comprising:
a housing assembly defining a first inner lumen axially extending therethrough;
a pusher assembly disposed within the first inner lumen, the pusher assembly comprising an inner core and an energy storage element, the inner core configured to be axially movable, the energy storage element disposed between the inner core and the housing assembly; and
a drive assembly,
wherein the drive assembly is configured to drive the inner core to move from distal to proximal to cause the energy storage element to store elastic potential energy, and the drive assembly is further configured to cause the energy storage element to release the stored elastic potential energy to drive the inner core to move from proximal to distal.

Optionally, the housing assembly may comprise a first locking member, wherein the pusher assembly further comprises a second locking member provided on the inner core, the second locking member configured to be maintained axially stationary with respect to the inner core and movable radially with respect to the inner core; and
wherein the drive assembly is configured to drive the inner core to move from distal to proximal until the second locking member is in engagement with the first locking member, and the drive assembly is further configured to drive the second locking member to move radially with respect to the inner core to disengage the second locking member from the first locking member and cause the energy storage element to release the elastic potential energy.

Optionally, the drive assembly may comprise a first drive member and a second drive member, the first drive member configured to drive the inner core to move from distal to proximal, the second drive member configured to drive the second locking member to move radially with respect to the inner core.

Optionally, the housing assembly may define an axially extending first guide slot in connection with the first inner lumen, wherein the first drive member comprises an engagement portion and a first manipulation portion, the engagement portion having an end connected to the inner core, and a further end inserted through the first guide slot to connect to the first manipulation portion.

Optionally, the first locking member may comprise a locking slot provided in the housing assembly, wherein the inner core comprises a radially extending first mounting hole, the first mounting hole aligned circumferentially with the locking slot; the second locking member is at least partially disposed in the first mounting hole and comprises a first elastic element and an ejector pin connected to the first elastic element;
when the ejector pin moves out from the first mounting hole into the locking slot, the second locking member is in engagement with the first locking member; and when the second drive member drives the ejector pin to radially move to compress the first elastic element until the ejector pin moves away from the locking slot, the second locking member is disengaged from the first locking member.

Optionally, two locking slots may be comprised, and the two locking slots are distributed in symmetry with respect to an axis of the housing assembly; the first mounting hole radially extending through the inner core; and wherein two ejector pins are comprised, and the two ejector pins are connected by the first elastic element, and each of the ejector pins defines a wedge-shaped actuation groove,
wherein the inner core defines an axially-extending second inner lumen which has an open proximal end and a distal end provided with a bottom wall defining therein an actuation channel in connection with the first mounting hole; the second drive member is at least partially disposed within the second inner lumen and configured to be axially movable in the second inner lumen; the second drive member distally defines two opposing wedge-shaped blocks complementary in shape to the respective actuation grooves; and
wherein the needle insertion assistant is configured such that when the second drive member moves from proximal to distal, the wedge-shaped blocks move through the actuation channel to engagement with the respective actuation grooves and drive the respective ejector pins to move towards one another so that the first elastic element is compressed.

Optionally, the actuation groove may define, at an edge thereof close to an axis of the inner core, a slanted surface, so that a distance from the slanted surface to the axis of the inner core gradually increases from proximal to distal.

Optionally, the slanted surface and the axis of the inner core may define therebetween an acute angle ranging from 5° to 45°.

Optionally, the second drive member may comprise a second manipulation portion and the wedge-shaped blocks connected to a distal end of the second manipulation portion, wherein the needle insertion assistant further comprises a second elastic element disposed between the distal end of the second manipulation portion and the bottom wall of the second inner lumen;
when the second drive member moves from proximal to distal, the second elastic element is compressed and stores elastic potential energy; and when the second elastic element releases the stored elastic potential energy, the second drive member is driven to move from distal to proximal.

Optionally, the needle insertion assistant may further comprise an axial stopper defining a maximum possible distance of axial movement of the second drive member.

Optionally, the axial stopper may comprise: an axially-extending second guide slot defined in a wall of the inner core; and a guide pin provided on the second drive member, the guide pin inserted through the second guide slot so as to be axially movable therein.

Optionally, when the second locking member is in engagement with the first locking member, a proximal end of the second drive member may protrude out of the proximal end of the second inner lumen, and a proximal end face of the second drive member may not protrude out of a proximal end face of the housing assembly, wherein the housing assembly defines two proximal notches in symmetry with respect to the axis of the housing assembly.

Optionally, the needle insertion assistant may further comprise a first circumferential stopper for maintaining the inner core to be circumferentially stationary with respect to the housing assembly

Optionally, an axially-extending mounting groove may be provided in a wall surface of the first inner lumen, and a corresponding protruding structure is provided on an outer surface of the inner core, wherein the energy storage element is disposed within the mounting groove so that an end of the energy storage element abuts against the wall surface of the mounting groove and a further end of the energy storage element abuts against the protruding structure.

Optionally, the housing assembly may comprise a housing body and a pre-tension adjustment mechanism, the housing body defining the first inner lumen, a first locating member on an outer surface of the housing body, the housing body defining an axially-extending third guide slot in connection with the first inner lumen, the pre-tension adjustment mechanism comprising a third drive member, a movable sleeve and a force transmission member, the third drive member rotatably sleeved over the outer surface of the housing body, the third drive member defining in an inner surface thereof a helical groove spiraling about an axis of the housing body, a second locating member formed on the third drive member, the movable sleeve disposed over the inner core, the force transmission member provided on an outer surface of the movable sleeve, the force transmission member inserted through the third guide slot into the helical groove,
wherein a protruding structure is provided on an outer surface of the inner core, and the energy storage element abuts at one end against the protruding structure and at a further end against the movable sleeve; and
the needle insertion assistant is configured so that when the third drive member is rotated by an external force about the axis of the housing body in a predetermined direction, the third drive member drives the movable sleeve to move from proximal to distal so that the energy storage element is compressed and stores elastic potential energy that provides a pre-tension and that when the external force is removed and the second locating member remains in engagement with the first locating member, the energy storage element is prevented from releasing the elastic potential energy.

Optionally, the first locating member may be a locating recess extending across an entire circumferential length of the housing body and having at least one wavy wall surface, wherein the second locating member is a stop block having a contour complementary in shape to the wall surface of the locating recess.

Optionally, the first locating member may comprise a plurality of locating recesses spaced apart circumferentially around the housing body, wherein the second locating member comprises a ball plunger, which, when inserted in one of the locating recesses, brings the second locating member into engagement with the first locating member.

Optionally, the housing assembly may further comprise a second circumferential stopper for maintaining the movable sleeve to be circumferentially stationary with respect to the housing body.

Optionally, the needle insertion assistant may further comprise an interface component connected to a distal end of the inner core and configured for connection with an object of interest.

To the above end, the present invention also provides a medical device comprising an object of interest and the needle insertion assistant as defined above, the object of interest comprising a needle, wherein a distal end of the inner core is configured for connection with the object of interest and for insertion of the needle to a target site.

Compared with the prior art, the needle insertion assistant and the medical device of the present invention have the advantages as follows:
The needle insertion assistant includes a housing assembly, a pusher assembly and a drive assembly. The housing assembly defines a first inner lumen axially extending therethrough. The pusher assembly is disposed within the first inner lumen and includes an inner core and an energy storage element. The inner core is configured to be axially movable and for distal connection with an object of interest including a needle. The energy storage element is disposed between the inner core and the housing assembly. In the needle insertion assistant, the drive assembly is configured to drive the inner core to move from distal to proximal to cause the energy storage element to deform and store elastic potential energy. The drive assembly is further configured to cause the energy storage element to release the stored elastic potential energy to drive the inner core to move from proximal to distal. As a result, perpendicular insertion of the needle to a target site can be achieved at a high speed without breakage of the needle.

### BRIEF DESCRIPTION OF THE DRAWINGS

The accompanying drawings are provided to facilitate a better understanding of the present invention and do not unduly limit the scope thereof in any sense, in which:
Fig. 1 is a schematic diagram showing the structure of a needle insertion assistant according to a first embodiment of the present invention;
Fig. 2 is a schematic exploded view of the needle insertion assistant according to the first embodiment of the present invention;
Fig. 3 is a partial cross-sectional view of the needle insertion assistant according to the first embodiment of the present invention, schematically showing how a second drive member is engaged with a pusher assembly, as well as a second locking member disposed distally with respect to a first locking member;
Fig. 4 is a partial cross-sectional view of the needle insertion assistant according to the first embodiment of the present invention, schematically showing how the second drive member is engaged with the pusher assembly, as well as how the second locking member is engaged with the first locking member;
Fig. 5 is a partial cross-sectional view of the needle insertion assistant according to the first embodiment of the present invention, schematically showing an arrangement of energy storage elements;
Fig. 6 is a partial cross-sectional view of the needle insertion assistant according to the first embodiment of the present invention, schematically showing an axial stopper;
Fig. 7 is a partial cross-sectional view of the needle insertion assistant according to the first embodiment of the present invention, schematically showing the axial stopper, as well as a guide pin differing from that of Fig. 6;
Fig. 8 is a schematic diagram showing the structure of a needle insertion assistant according to a second embodiment of the present invention;
Fig. 9 is a schematic exploded view of the needle insertion assistant according to the second embodiment of the present invention;
Fig. 10 is a schematic diagram showing the structure of part of the needle insertion assistant according to the second embodiment of the present invention;
Fig. 11 is a schematic exploded view of a needle insertion assistant according to a third embodiment of the present invention; and
Fig. 12 is a schematic diagram showing the structure of part of the needle insertion assistant according to the third embodiment of the present invention.

List of Reference Numerals in Drawings
1001 - First Inner Lumen; 1002 - First locking member; 1003 - First Guide Slot; 1004 - Notch; 1005 - Mounting Groove; 1100 - Housing Body; 1101 - First Locating Member; 1102 - Third Guide Slot; 1200 - Pre-tension Adjustment Mechanism; 1210 - Third Drive Member; 1211 - Helical Groove; 1212 - Second Locating Member; 1220 - Movable Sleeve; 1221 - Second Raised Structure; 1230 - Force Ttransmission Member;
2000 - Pusher Assembly; 2100 - Inner Core; 2101 - First Mounting Hole; 2102 - Second Inner Lumen; 2103 - Second Guide Slot; 2110 - Protruding Structure; 2111 - Annular Protrusion; 2112 - First Raised Structure; 2200 - Energy Storage Element; 2300 - Second Locking Member; 2310 - First Elastic Element; 2320 - Ejector Pin; 2321 - Actuation Groove; 2322 - Pin Body; 2323 - Insertion Section;
3100 - First Drive Member; 3110 - Engagement Portion; 3120 - First Manipulation Portion; 3200 - Second Drive Member; 3210 - Wedge-Shaped Block; 3220 - Guide Pin; 3221 - Third Elastic Element; 3222 - Fastener; 3230 - Second Manipulation Portion;
4000 - Interface Component;
5000 - Second Elastic Element.

### DETAILED DESCRIPTION

Particular embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. Throughout these drawings, like numerals indicate like elements.

As used herein, in context of relative orientations, relative positions and directions between components of a medical device or actions thereof, as viewed by a physician operating the medical device, without wishing to be limiting, the term "distal end" usually refers to an end of the device closer to a patient, and "proximal end" refers to an end thereof opposite to the distal end.

### EMBODIMENT 1

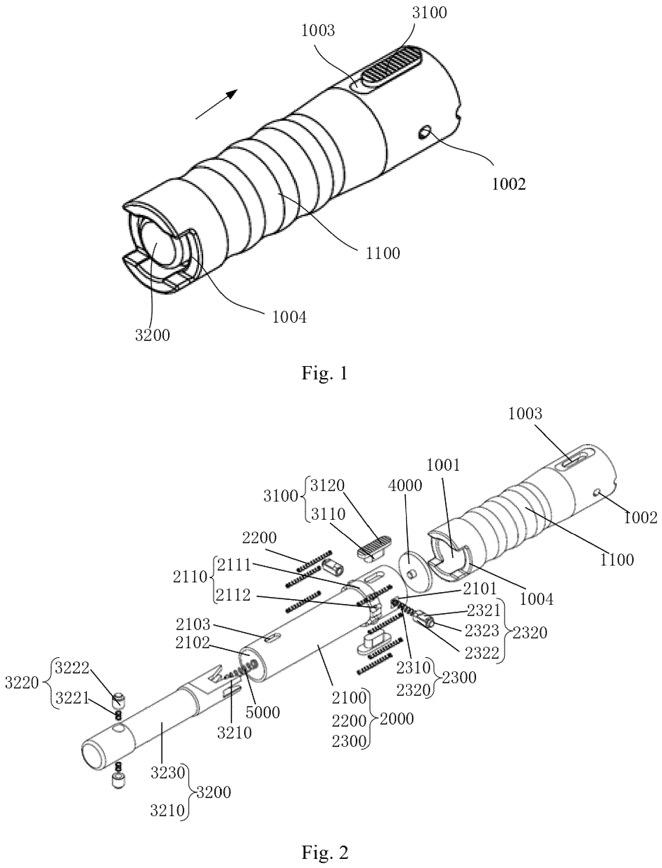

Fig. 1 is a schematic diagram showing the structure of a needle insertion assistant according to a first embodiment of the present invention, and Fig. 2 is a schematic exploded view thereof.

Referring to Figs. 1 and 2, the needle insertion assistant includes a housing assembly, a pusher assembly 2000 and a drive assembly. The housing assembly defines a first inner lumen 1001 axially extending therethrough. The pusher assembly 2000 is disposed within the first inner lumen 1001 and includes an inner core 2100 and an energy storage element 2200. The inner core 2100 is configured to be movable in an axial direction of the needle insertion assistant. The energy storage element 2200 is disposed between the inner core 2100 and the housing assembly. The needle insertion assistant is configured so that the drive assembly can drive the inner core 2100 to move from distal to proximal and thereby cause the energy storage element 2200 to deform and store elastic potential energy. The drive assembly is also used to cause the energy storage element 2200 to release the elastic potential energy and thereby drive the pusher assembly 2000 to move from proximal to distal (i.e., in the direction indicated by the arrow in Fig. 1).

The inner core 2100 is configured t0 distal connection with an object of interest (not shown) including, for example, a needle extending in the axial direction of the needle insertion assistant. During use, a user may first actuate the drive assembly to cause the energy storage element 2200 to store elastic potential energy and then again actuate the drive assembly to cause the energy storage element 2200 to release the elastic potential energy, resulting in fast movement of the inner core 2100 together with the object from proximal to distal and hence perpendicular insertion of the needle into a target site without breakage of the needle. Those skilled in the art would recognize that the target site may be subcutaneous in a patient and that the needle may be a pointed protrusion capable of piercing the skin and reaching a location thereunder. Examples of the object of interest may include, but are not limited to, microneedle patches, needle assemblies in drug (e.g., insulin) infusion systems and blood glucose monitoring devices, and the like. It is to be noted that the needle insertion assistant has generally a cylindrical structure defining a lengthwise direction (i.e., axial direction) and a radial direction perpendicular to the axial direction. As used herein, the terms "axial direction", "axially", "radial direction" and "radially" generally all relate to the needle insertion assistant, unless the context clearly dictates otherwise.

In greater detail, the housing assembly is provided with a first locking member 1002, and the pusher assembly 2000 includes a second locking member 2300 provided on the inner core 2100. The second locking member 2300 is configured so that it remains stationary axially with respect to the inner core 2100 while being movable radially with respect to the inner core 2100. Moreover, the drive assembly includes a first drive member 3100 and a second drive member 3200. The needle insertion assistant is configured so that the first drive member can drive the inner core 2100 to move from distal to proximal to bring the second locking member 2300 into engagement with the first locking member 1002, allowing the energy storage element 2200 to maintain elastic potential energy that it stores. The second drive member 3200 is used to drive the second locking member 2300 to move radially with respect to the inner core 2100 to disengage the second locking member 2300 from the first locking member 1002, allowing the energy storage element 2200 to release the stored elastic potential energy. That is, during practical use, the user can actuate the first drive member 3100 first and then actuate the second drive member 3200.

Optionally, as shown in Fig. 2, the needle insertion assistant may further include an interface component 4000, through which the object of interest is connected to the distal end of the inner core 2100. Those skilled in the art would recognize that the interface component 4000 may be structured depending on the type of the object of interest, as long as it allow the distal connection of the inner core 2100 with the object of interest. The interface component 4000 may be connected to the distal end of the inner core 2100 by another suitable means such as snap fasteners, an interference fit, a threaded connection or gluing.

Structural details of the housing assembly, the pusher assembly 2000 and the drive assembly of the needle insertion assistant, as well as how they are assembled together, will be explained below.

With continued reference to Fig. 1, in conjunction with Fig. 2, the housing assembly may have a circular, triangular, rectangular, polygonal or another cross-sectional shape. This embodiment is exemplified in the context of the housing assembly having a circular cross-section.

In this embodiment, the housing assembly includes a housing body 1100 defining the first inner lumen 1001. A wall of the housing body 1100 defines a first guide slot 1003 in connection with the first inner lumen 1001. The first guide slot 1003 is an elongate slot and may be defined around a distal end of the housing body 1100.

The inner core 2100 has a cross-section matching that of the housing body 1100. That is, the inner core 2100 may also be a cylindrical structure. Preferably, the inner core 2100 is arranged within the first inner lumen 1001 so as to be coaxial therewith. The inner core 2100 is coupled to the housing body 1100 by the first drive member 3100. Specifically, the first drive member 3100 includes an engagement portion 3110 and a first manipulation portion 3120. One end of the engagement portion 3110 is attached to the inner core 2100, and the other end of engagement portion 3110 passes through the first guide slot 1003 to the outside of the housing body 1100 and joined to the first manipulation portion 3120. The first manipulation portion 3120 is configured to receive an external force and move axially within the first guide slot 1003 under the action of the external force, thereby driving the inner core 2100 to move from distal to proximal. Those skilled in the art would appreciate that the first drive member 3100 can axially move a maximum possible distance that is dependent on a length of the first guide slot 1003, and the inner core 2100 can axially move a maximum possible distance that is in turn dependent on said maximum possible distance of the first drive member 3100. Preferably, there are two first guide slots 1003, which are distributed in symmetry with respect to an axis of the housing body 1100, and accordingly two first drive members 3100 each having its engagement portion 3110 passed through a respective one of the first guide slots 1003 and attached to the inner core 2100. Providing the two first drive members 3100 enables more even stressing and smoother movement of the inner core 2100.

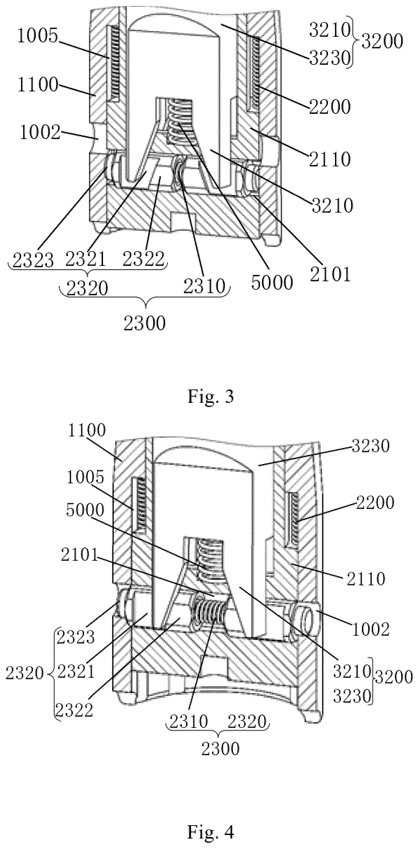

Referring to Fig. 3, a mounting groove 1005, which extends axially and is distally open, is provided in an inner wall surface of the housing body 1100 (i.e., a wall surface of the first inner lumen 1001). The energy storage element 2200 may be mounted in the mounting groove 1005. Preferably, a plurality of such mounting grooves 1005 are provided circumferentially in symmetry around the housing body 1100 about its axis. A protruding structure 2110 corresponding to the mounting groove 1005 is provided on an outer surface of the inner core 2100.

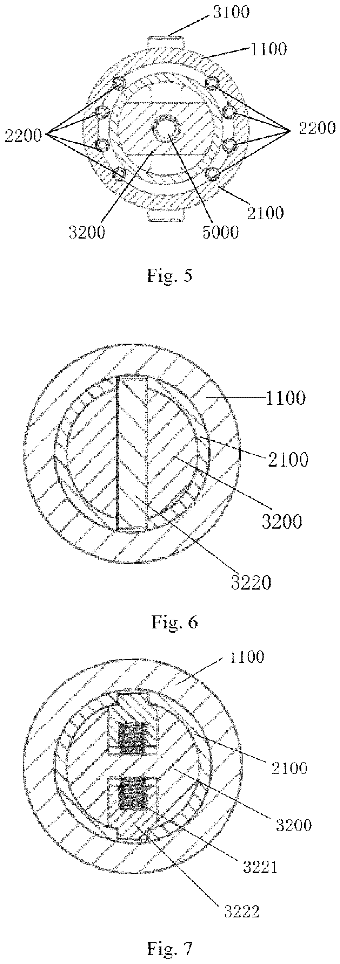

The energy storage element 2200 may be an elastic element such as a spring defining an axis parallel to the axis of the needle insertion assistant. The energy storage element 2200 is mounted in the mounting groove 1005 so that its one end abuts against a wall surface of the mounting groove 1005 and its other end abuts against the protruding structure 2110. Preferably, there are a plurality of energy storage elements 2200 each mounted in a respective of corresponding mounting grooves 1005 so that the energy storage elements 2200 are distributed in symmetry about the axis of the needle insertion assistant (see Fig. 5). In this way, the needle insertion assistant can be adapted to various types of objects of interest. The number of energy storage elements 2200 may be determined as actually needed, and those skilled in the art would appreciate that more energy storage elements 2200 can store more elastic potential energy which means a larger pushing force for driving the inner core 2100 to move from proximal to distal. This can facilitate quick transdermal insertion of a needle and is particular useful in case of the object of interest being implemented as a microneedle patch.

The needle insertion assistant further includes a first circumferential stopper for keeping the inner core 2100 circumferentially stationary with respect to the housing assembly. In a preferred implementation, as shown in Fig. 2, the protruding structure 2110 includes an annular protrusion 2111 and a first raised structure 2112. The annular protrusion 2111 extends across the entire circumferential length of the inner core 2100, and the first raised structure 2112 is provided on the annular protrusion 2111. The first raised structure 2112 is at least partially received in the mounting groove 1005. On the one hand, this can enable the abutment of the energy storage element 2200. On the other hand, the first raised structure 2112 and the mating mounting groove 1005 can together form the first circumferential stopper (i.e., the first circumferential stopper comprises the first raised structure 2112 and the mounting groove 1005), adding compactness to the structure of the needle insertion assistant. Those skilled in the art would recognize that the protruding structure 2110 and the inner core 2100 may be integrally formed, or separately fabricated and then joined to either by welding, gluing or another other suitable approach.

Used as the first locking member 1002, a locking slot may be provided in the housing body 1100. Optionally, two locking slots are provided in symmetry with respect to the axis of the housing body 1100. The inner core 2100 defines a first mounting hole 2101 preferably extending through the needle insertion assistant radially. Opposite ends of the first mounting hole 2101 may be aligned with the respective two locking slots circumferentially about the needle insertion assistant. The second locking member 2300 is at least partially disposed in the first mounting hole 2101 and includes a first elastic element 2310 and an ejector pin 2320. Preferably, two ejector pins 2320 are disposed at opposite ends of the first elastic element 2310 and able to extend outwardly from the first mounting hole 2101.

Before the first drive member 3100 is actuated, the first mounting hole 2101 and the second locking member 2300 mounted therein are located distally with respect to the locking slot, and the ejector pin 2320 presses against the wall surface of the first inner lumen 1001 so that the first elastic element 2310 is compressed and stores elastic potential energy. As the first drive member 3100 drives the inner core 2100 to move from distal to proximal, the second locking member 2300 moves therewith toward the locking slot. Upon the second locking member 2300 reaching the locking slot, the ejector pin 2320 will no longer press against the wall surface of the first inner lumen 1001, and the first elastic element 2310 will release the stored elastic potential energy and lengthen, causing extension of the ejector pin 2320 from the first mounting hole 2101 into the locking slot and thereby locking the second locking member 2300 to the first locking member 1002. On the contrary, as a result of the second drive member 3200 driving the ejector pin 2320 to radially move relative to the needle insertion assistant to compress the first elastic element 2310 until the ejector pin 2320 moves away from the locking slot, the second locking member 2300 can be unlocked from the first locking member 1002. Examples of the first elastic element 2310 may include, but are not limited to, springs.

In greater detail, with particular reference to Figs. 2 to 4, the inner core 2100 defines an axially-extending second inner lumen 2102 proximal with respect to the first mounting hole 2101. The second inner lumen 2102 is proximally open and has a distal bottom wall defining an actuation channel in connection with the second inner lumen 2102. The ejector pin 2320 defines a wedge-shaped actuation groove 2321. The second drive member 3200 is at least partially disposed in the second inner lumen 2102 and configured to be axially movably under the action of an external force. The second drive member 3200 defines at its distal end two wedge-shaped blocks 3210 complementary in shape to the actuation groove 2321. When the second drive member 3200 is driven by an external force to move from proximal to distal, the wedge-shaped blocks 3210 will pass through the actuation channel, come into engagement with the actuation grooves 2321 and cause movement of the ejector pins 2320 until the first elastic element 2310 is compressed and the ejector pins 2320 move away from the locking slot.

In this embodiment, the actuation groove 2321 defines a slanted surface at its side closer to an axis of the inner core 2100 such that a distance from the slanted surface to the axis of the inner core 2100 gradually increases from proximal to distal. The slanted surface and the axis of the inner core 2100 define therebetween an acute angle in the range of 5° to 45°, such as 20°.

Preferably, the ejector pin 2320 includes a pin body 2322 and an insertion section 2323. The pin body 2322 defines the actuation groove 2321 and has a cross-sectional area greater than a cross-sectional area of the locking slot, while the insertion section 2323 has a cross-sectional area smaller than that of the locking slot. In this way, the insertion section 2323 can be inserted into the locking slot, and the second locking member 2300 will not move away from the inner core 2100 and the housing body 1100 through the locking slot. In addition, in order to prevent rotation of the ejector pin 2320 about its own axis, it, and accordingly the locking slot, is preferred to have a non-circular cross-sectional shape.

The needle insertion assistant further includes an axial stopper defining a maximum possible distance of axial movement of the second drive member 3200. In a non-limiting embodiment as shown in Figs. 6 and 7, the axial stopper includes a second guide slot 2103 provided in a wall of the inner core 2100 and a guide pin 3220 provided on the second drive member 3200. The second guide slot 2103 is an axially elongate slot, and the guide pin 3220 is inserted in the second guide slot 2103 so as to be axially movable therein. In this way, the maximum possible distance of axial movement of the second drive member 3200 depends on a length of the second guide slot 2103.

Optionally, there are two second guide slots 2103 defined in symmetry circumferentially around the inner core 2100. The guide pin 3220 may be a pin (see Fig. 6) long enough to be radially inserted through the second drive member 3200, with its opposite ends being received in the respective second guide slots 2103. Alternatively, two shorter guide pins 3220 may be used, each of which is coupled to the second drive member 3200, with its end away from the second drive member 3200 being received in a respective one of the second guide slots 2103. Still alternatively, the second drive member 3200 may define two second mounting holes (not labeled) in symmetry with respect to an axis of the second drive member 3200. Moreover, the guide pins 3220 comprises two spring pins (see Figs. 2 and 7) each including a third elastic element 3221 and a fastener 3222 secured within one of the second mounting holes by the third elastic element 3221, with an end of the fastener 3222 away from the third elastic element 3221 being received in a respective one of the second guide slots 2103. The guide pins 3220 and the two second guide slots 2103 can work together to make stressing of the guide pins 3220 more even.

Referring back to Fig. 2, in conjunction with Figs. 3 and 4, the second drive member 3200 includes a second manipulation portion 3230 and the wedge-shaped block 3210 joined to a distal end of the second manipulation portion 3230. The needle insertion assistant further includes a second elastic element 5000, which may be implemented as, for example, a spring, and is disposed between the distal end of the second manipulation portion 3230 and the bottom wall of the second inner lumen 2102. When the second locking member 2300 is unlocked from the first locking member 1002 as a result of movement of the second drive member 3200 from proximal to distal under the action of an external force, the second elastic element 5000 is compressed and stores elastic potential energy. When the external force is removed, the second elastic element 5000 will release the stored elastic potential energy and thereby cause the second drive member 3200 to move from distal to proximal to its rest position.

In addition, referring to Fig. 1, in the configuration where the second locking member 2300 is locked to the first locking member 1002, the second drive member 3200 proximally protrudes out of the proximal end of the second inner lumen 2102, but a proximal end face of the second drive member 3200 is not located proximally beyond a proximal end face of the housing body 1100 (i.e., the proximal end face of the second drive member 3200 is located proximally with respect to a proximal end face of the inner core 2100 and is aligned with, or located distally with respect to, the proximal end face of the housing body 1100). Correspondingly, the housing body 1100 defines two proximal notches 1004 in symmetry with respect to the axis of the housing body 1100, which allow the user to conveniently apply an external force to the second drive member 3200 to cause its axial movement and can prevent incorrect operation to a certain extent.

Use of the needle insertion assistant can begin with attachment of the object of interest to the distal end of the inner core 2100 through the interface component 4000. The user may then apply an external force to the first manipulation portion 3120 to cause the first drive member 3100 to drive the inner core 2100 to move from distal to proximal to allow the energy storage element 2200 to store elastic potential energy until the second locking member 2300 comes into locking engagement with the first locking member 1002. Subsequently, the needle insertion assistant may be distally aligned with an intended portion of a patient's body surface so that a needle in the object is oriented perpendicularly to the body surface portion. After that, the user may apply another external force to the second manipulation portion 3230 from the notches 1004 of the housing assembly to drive the second drive member 3200 to move from proximal to distal to unlock the second locking member 2300 from the first locking member 1002. As a result, the energy storage element 2200 releases the stored elastic potential energy and thereby pushes the inner core 2100 with the object of interest retained thereon, leading to fast movement thereof from proximal to distal and subcutaneous insertion of the needle. At the same time, the first drive member 3100 returns back to its rest position, and the second drive member 3200 is also biased back to its rest position by the second elastic element 5000. Finally, the object of interest may be detached from the interface component 4000.

The needle insertion assistant provided in this embodiment can be held and operated by the user with a single hand and is therefore easy and simple to use. This needle insertion assistant is robust and can provide a large pushing force by employing a plurality of energy storage elements, which can ensure effective subcutaneous insertion of the object of interest.

Also provided in this embodiment is a medical device including the above-discussed needle insertion assistant and object of interest that can be attached to the distal end of the inner core.

### EMBODIMENT 2

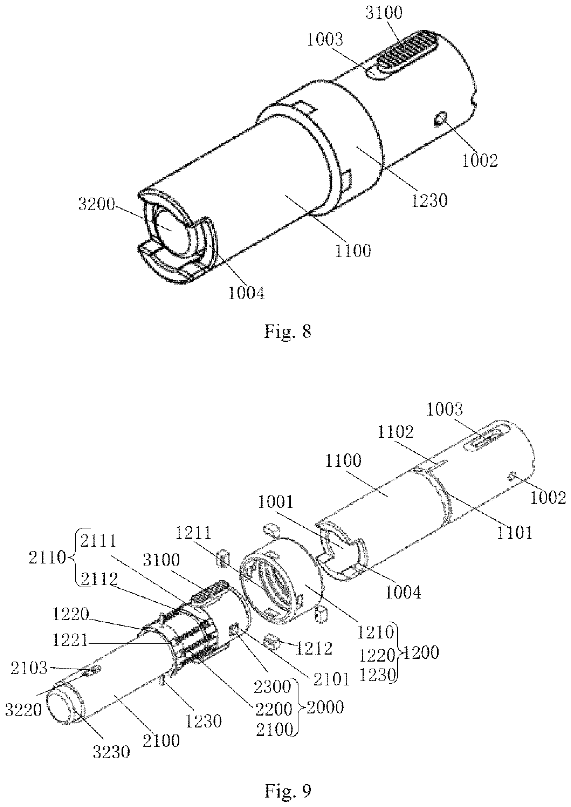

Referring to Figs. 8 to 10, a second embodiment differs from the first embodiment in that the housing assembly further includes a pre-tension adjustment mechanism 1200 in addtion to the housing body 1100.

Specifically, the housing body 1100 defines, on its outer surface, a first locating member 1101 and a third guide slot 1102. The third guide slot 1102 is in connection with the first inner lumen 1001 and extends axially. The third guide slot 1102 is preferably between the first locating member 1101 and the first guide slot 1003. The pre-tension adjustment mechanism 1200 includes a third drive member 1210, a movable sleeve 1220 and a force transmission member 1230. The third drive member 1210 is disposed over the housing body 1100, kept axially stationary with respect to the housing body 1100 and circumferentially rotatable around the housing body 1100. In an inner surface of the third drive member 1210, there is provided a helical groove 1211 spiraling about the axis of the housing body 1100. The helical groove 1211 may have a rectangular or trapezoidal cross-sectional shape. The third drive member 1210 defines a second locating member 1212 for engagement and cooperation with the first locating member 1101. The movable sleeve 1220 is disposed within the first inner lumen 1001 and over the inner core 2100 and configured to be axially movable. The force transmission member 1230 is attached to an outer surface of the movable sleeve 1220 and inserted through the third guide slot 1102 into the helical groove 1211.

Further, the aforementioned mounting groove on the inner surface of the housing body 1100 is omitted. Instead, one end of the energy storage element 2200 abuts against the movable sleeve 1220, and the other end abuts against the protruding structure 2110.

In this embodiment, before the first drive member 1210 is actuated, when rotated by an external force in a predetermined direction about the axis of the housing body 1100, the third drive member 1210 drives, through the force transmission member 1230, the movable sleeve 1220 to move from proximal to distal in the axial direction of the needle insertion assistant so that the energy storage element is compressed and stores elastic potential energy that provides a pre-tension. When the external force is removed and the second locating member 1212 is locked to the first locating member 1101, the pre-tension provided by the energy storage element stops increasing. The predetermined direction may be either clockwise or counterclockwise, as actually needed. The pre-tension may be adjusted by changing the number of revolutions that the second drive member 1210 has made.

Optionally, two force transmission members 1230 may be provided at an angular interval of 180° circumferentially around the needle insertion assistant and an axial interval equal to a 0.5 times a pitch of the helical groove 1211 in the axial direction of the needle insertion assistant. In this way, the movable sleeve 1220 will stressed more even and will not tilt. The second locating member 1212 and the third drive member 1210 may be fabricated separately and then coupled together by an interference fit (in this case, the third drive member 1210 further defines a third mounting hole), adhesive bond, fastener or any other suitable means.

Optionally, the first locating member 1101 may be a loacting recess extending across the entire circumferential length of the housing body 1100, and at least one wall surface of the loacting recess may have a wavy shape. The second locating member 1212 may be a stop block having a contour complementary in shape to the wall surface of the loacting recess. The user may turn the third drive member 1210 to cause the stop block slide over the wavy wall surface of the loacting recess and, in this process, can feel resistance to the sliding by hand sensation.

In this embodiment, the needle insertion assistant may also include a first circumferential stopper optionally including a first raised structure 2112 on the protruding structure 2110 and a first groove (not labeled) in the inner surface of the housing body 1100. Apart from this, the needle insertion assistant may further include a second circumferential stopper for maintaining the movable sleeve 1220 circumferentially stationary with respect to the housing body 1100. A second raised structure 1221 may be provided on the movable sleeve 1220, and a mating second groove (not labeled) may be provided in the inner surface of the housing body 1100. When the second raised structure 1221 is at least partially received in the second groove, they together form the second circumferential stopper. Those skilled in the art would appreciate that when the first raised structure 2112 and the second raised structure 1221 are aligned with each other circumferentially with respect to the needle insertion assistant, the first and second grooves may come into connection with each other. Further, the energy storage element 2200 may abut at its opposite ends respectively against the first raised structure 2112 and the second raised structure 1221.

Use of the needle insertion assistant can begin with attachment of the object of interest to the distal end of the inner core 2100 through the interface component 4000. The third drive member 1210 may be then rotated to cause the energy storage element 2200 to store elastic potential energy and provide a pre-tension. After that, the first drive member 3100 may be actuated to additionally deform the energy storage element 2200 and allow it to store more elastic potential energy. Subsequently, a needle in the object may be oriented perpendicularly to an intended portion of a patient's body surface, and the second drive member 3200 may be actuated to cause the energy storage element 2200 to release all the stored elastic potential energy to achieve subcutaneous insertion of the needle. Finally, the object of interest may be detached from the interface component 4000. Compared with the first embodiment, for a given number of energy storage elements 2200 being used, these energy storage elements 2200 can be deformed more profoundly to provide an even larger pushing force, which enables a greater impact on the needle and thus allows its faster subcutaneous insertion. Thus, this embodiment is particularly useful with objects of interest requiring a greater pushing force.

Also provided in this embodiment is a medical device including the above-discussed needle insertion assistant and object of interest that can be attached to the distal end of the inner core.

### EMBODIMENT 3

A third embodiment differs from the second embodiment in that first locating member 1101 and the second locating member 1212 structured differently.

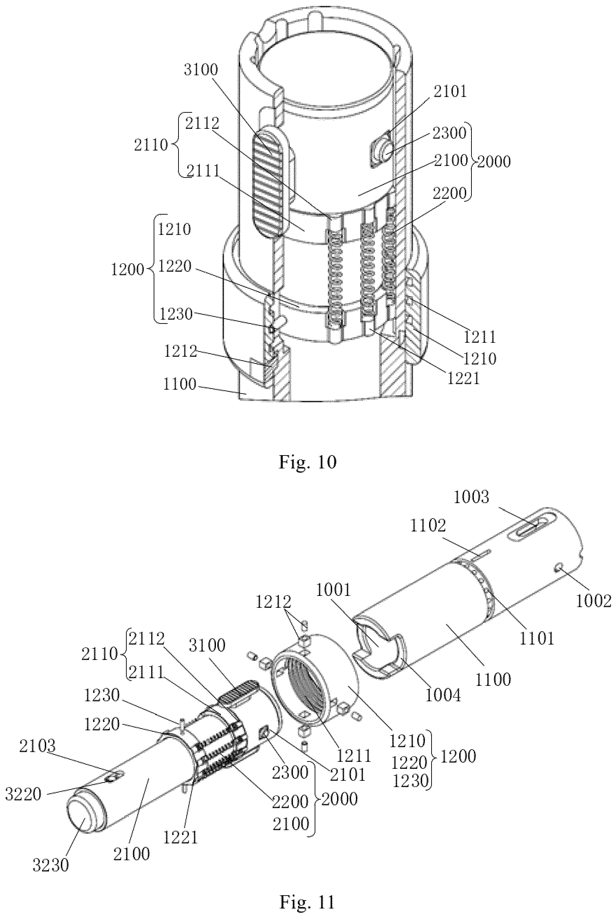

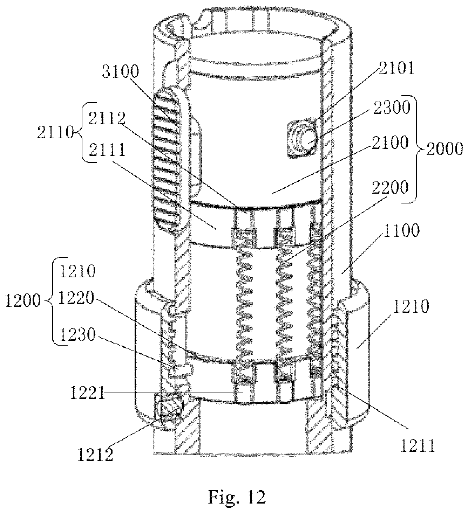

As shown in Figs. 11 and 12, in this embodiment, the first locating member 1101 includes a plurality of dome-shaped locating recesses spaced apart circumferentially around the housing body 1100. The second locating member 1212 is a ball plunger. Those skilled in the art would recognize that a ball plunger has a structure known in the art, which typically composed of a compression spring and a steel ball attached to the compression spring. When the second locating member 1212 is brought into alignment with one of the locating recesses as a result of rotation of the third drive member 1210, the steel ball will be pushed by the compression spring into the locating recesses, locking the second locating member 1212 to the first locating member 1101. The ball plunger may be connected to the third drive member 1210 by a locating block. Additionally, the locating recesses may be defined in a stop groove which is formed in an outer surface of the housing body 1100 so as to extend across its entire circumferential length.

Also provided in this embodiment is a medical device including the above-discussed needle insertion assistant and object of interest that can be attached to the distal end of the inner core.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A needle insertion assistant, comprising:
a housing assembly defining a first inner lumen axially extending therethrough;
a pusher assembly disposed within the first inner lumen, the pusher assembly comprising an inner core and an energy storage element, the inner core configured to be axially movable, the energy storage element disposed between the inner core and the housing assembly; and
a drive assembly,
wherein the drive assembly is configured to drive the inner core to move from distal to proximal to cause the energy storage element to store elastic potential energy, and the drive assembly is further configured to cause the energy storage element to release the stored elastic potential energy to drive the inner core to move from proximal to distal.

2. The needle insertion assistant according to claim 1, wherein the housing assembly comprises a first locking member, wherein the pusher assembly further comprises a second locking member provided on the inner core, the second locking member configured to be maintained axially stationary with respect to the inner core and movable radially with respect to the inner core; and
wherein the drive assembly is configured to drive the inner core to move from distal to proximal until the second locking member is in engagement with the first locking member, and the drive assembly is further configured to drive the second locking member to move radially with respect to the inner core to disengage the second locking member from the first locking member and cause the energy storage element to release the elastic potential energy.

3. The needle insertion assistant according to claim 2, wherein the drive assembly comprises a first drive member and a second drive member, the first drive member configured to drive the inner core to move from distal to proximal, the second drive member configured to drive the second locking member to move radially with respect to the inner core.

4. The needle insertion assistant according to claim 3, wherein the housing assembly defines an axially extending first guide slot in connection with the first inner lumen, wherein the first drive member comprises an engagement portion and a first manipulation portion, the engagement portion having an end connected to the inner core, and a further end inserted through the first guide slot to connect to the first manipulation portion.

5. The needle insertion assistant according to claim 3, wherein the first locking member comprises a locking slot provided in the housing assembly, wherein the inner core comprises a radially extending first mounting hole, the first mounting hole aligned circumferentially with the locking slot; the second locking member is at least partially disposed in the first mounting hole and comprises a first elastic element and an ejector pin connected to the first elastic element;
when the ejector pin moves out from the first mounting hole into the locking slot, the second locking member is in engagement with the first locking member; and when the second drive member drives the ejector pin to radially move to compress the first elastic element until the ejector pin moves away from the locking slot, the second locking member is disengaged from the first locking member.

6. The needle insertion assistant according to claim 5, wherein two locking slots are comprised, and the two locking slots are distributed in symmetry with respect to an axis of the housing assembly; the first mounting hole radially extending through the inner core; and wherein two ejector pins are comprised, the two ejector pins are connected by the first elastic element, and each of the ejector pins defines a wedge-shaped actuation groove,
wherein the inner core defines an axially-extending second inner lumen which has an open proximal end and a distal end provided with a bottom wall defining therein an actuation channel in connection with the first mounting hole; the second drive member is at least partially disposed within the second inner lumen and configured to be axially movable in the second inner lumen; the second drive member distally defines two opposing wedge-shaped blocks complementary in shape to the respective actuation grooves; and
wherein the needle insertion assistant is configured such that when the second drive member moves from proximal to distal, the wedge-shaped blocks move through the actuation channel to engage with the respective actuation grooves and drive the respective ejector pins to move towards one another so that the first elastic element is compressed.

7. The needle insertion assistant according to claim 6, wherein the actuation groove defines, at an edge thereof close to an axis of the inner core, a slanted surface, so that a distance from the slanted surface to the axis of the inner core gradually increases from proximal to distal.

8. The needle insertion assistant according to claim 7, wherein the slanted surface and the axis of the inner core define therebetween an acute angle ranging from 5° to 45°.

9. The needle insertion assistant according to claim 6, wherein the second drive member comprises a second manipulation portion and the wedge-shaped blocks, the wedge-shaped blocks connected to a distal end of the second manipulation portion, wherein the needle insertion assistant further comprises a second elastic element disposed between the distal end of the second manipulation portion and the bottom wall of the second inner lumen;
when the second drive member moves from proximal to distal, the second elastic element is compressed and stores elastic potential energy; and when the second elastic element releases the stored elastic potential energy, the second drive member is driven to move from distal to proximal.

10. The needle insertion assistant according to claim 6, further comprising an axial stopper defining a maximum possible distance of axial movement of the second drive member.

11. The needle insertion assistant according to claim 10, wherein the axial stopper comprises: an axially-extending second guide slot defined in a wall of the inner core; and a guide pin provided on the second drive member, the guide pin inserted through the second guide slot so as to be axially movable therein.

12. The needle insertion assistant according to claim 6, wherein when the second locking member is in engagement with the first locking member, a proximal end of the second drive member protrudes out of the proximal end of the second inner lumen, and a proximal end face of the second drive member does not protrude out of a proximal end face of the housing assembly, wherein the housing assembly defines two proximal notches in symmetry with respect to the axis of the housing assembly.

13. The needle insertion assistant according to any of claims 1 to 12, further comprising a first circumferential stopper for maintaining the inner core to be circumferentially stationary with respect to the housing assembly.

14. The needle insertion assistant according to claim 1, wherein an axially-extending mounting groove is provided in a wall surface of the first inner lumen, and a corresponding protruding structure is provided on an outer surface of the inner core, wherein the energy storage element is disposed within the mounting groove so that an end of the energy storage element abuts against the wall surface of the mounting groove and a further end of the energy storage element abuts against the protruding structure.

15. The needle insertion assistant according to claim 1, wherein the housing assembly comprises a housing body and a pre-tension adjustment mechanism, the housing body defining the first inner lumen, a first locating member provided on an outer surface of the housing body, the housing body defining an axially-extending third guide slot in connection with the first inner lumen, the pre-tension adjustment mechanism comprising a third drive member, a movable sleeve and a force transmission member, the third drive member rotatably sleeved over the outer surface of the housing body, the third drive member defining in an inner surface thereof a helical groove spiraling about an axis of the housing body, a second locating member formed on the third drive member, the movable sleeve disposed over the inner core, the force transmission member provided on an outer surface of the movable sleeve, the force transmission member inserted through the third guide slot into the helical groove,
wherein a protruding structure is provided on an outer surface of the inner core, and the energy storage element abuts at one end against the protruding structure and at a further end against the movable sleeve; and
the needle insertion assistant is configured so that when the third drive member is rotated by an external force about the axis of the housing body in a predetermined direction, the third drive member drives the movable sleeve to move from proximal to distal so that the energy storage element is compressed and stores elastic potential energy that provides a pre-tension and that when the external force is removed and the second locating member remains in engagement with the first locating member, the energy storage element is prevented from releasing the elastic potential energy.

16. The needle insertion assistant according to claim 15, wherein the first locating member is a locating recess extending across an entire circumferential length of the housing body and having at least one wavy wall surface, wherein the second locating member is a stop block having a contour complementary in shape to the wall surface of the locating recess.

17. The needle insertion assistant according to claim 15, wherein the first locating member comprises a plurality of locating recesses spaced apart circumferentially around the housing body, wherein the second locating member comprises a ball plunger, which, when inserted in one of the locating recesses, brings the second locating member into engagement with the first locating member.

18. The needle insertion assistant according to claim 15, wherein the housing assembly further comprises a second circumferential stopper for maintaining the movable sleeve to be circumferentially stationary with respect to the housing body.

19. The needle insertion assistant according to claim 1, further comprising an interface component connected to a distal end of the inner core and configured for connection with an object of interest.

20. A medical device, comprising an object of interest and the needle insertion assistant according to any of claims 1 to 19, the object of interest comprising a needle, wherein a distal end of the inner core is configured for connection with the object of interest and for insertion of the needle to a target site.
